# EUROPÄISCHE PATENTANMELDUNG

(11) **EP 0 684 024 A2**
(43) Veröffentlichungstag der Anmeldung: **29.11.1995**
(21) Anmeldenummer: 95810265.9
(22) Anmeldetag: 21.04.1995
(51) Int. Cl.: A61F 2/42

(54) **Gelenkprothese**

(30) Priorität: 26.04.1994 DE 4414426
(71) Anmelder: Cserhati, Zsuzsa, CH-8004 Zürich (CH)
(72) Erfinder: Segmüller, Gottfried Dr. med., CH-9000 St. Gallen (CH); Sennwald, Gontran Dr. med., CH-9000 St. Gallen (CH); Cserhati, Zsuzsa, CH-8004 Zürich (CH); Schaap, Gerard R. Dr.med, NL-1222 SZ Hilversum (NL)
(74) Vertreter: Rottmann, Maximilian R.

(57) **Zusammenfassung**

Eine Gelenkprothese weist zwei zusammenfügbare, zusammenwirkende Prothesenhälften (1, 10) mit je einem Verankerungsschaft (4, 13) zum jeweiligen Verankern in einem der Gelenkprothese zugewandten Knochen auf. Die erste Prothesenhälfte (1) verfügt über einen im wesentlichen kugelförmigen Kopf (2), der zum Zusammenfügen mit der zweiten Prothesenhälfte (10) mit diametral gegenüberliegenden Abflachungen (3b) versehen ist. Die zweite Prothesenhälfte (10) weist eine im wesentlichen kugelkalottenförmig ausgestaltete Pfanne (11) auf, deren Höhe grösser als der Kugalradius ist und die mit einem Ausschnitt zum Zusammenfügen der beiden Prothesenhälften (1, 10) in einer Montagestellung versehen ist. Zum Zusammenfügen werden die beiden Prothesenhälften (1, 10) in die Montagestellung ausgerichtet und nach dem Zusammenfügen in eine Wirkstellung gebracht, indem die eine gegenüber der anderen Prothesenhälfte (1, 10) verdreht wird. In dieser Wirkstellung ist die Gelenkprothese in einem Arbeitsbereich auf Zug belastbar. Mit einer solchen Gelenkprothese wird die Luxationsgefahr gegenüber bekannten Gelenkprothesen minimiert, wobei gleichzeitig eine hohe Beweglichkeit gewährleistet ist.

## Beschreibung

Bekannte Gelenkprothesen bestehen im allgemeinen aus zwei zusammenfügbaren, zusammenwirkenden Prothesenteilen. Der eine Prothesenteil weist üblicherweise eine konkav ausgebildete Pfanne auf, in welcher der andere Prothesenteil mit seinem Kopf beweglich abgestützt werden kann. Auf diese Weise wird ein Gelenk gebildet, welches die physiologischen Bewegungen nachbilden kann. Nachteilig bei diesen bekannten Gelenkprothesen ist, dass sie nur Druckkräfte jedoch keine Zugkräfte aufnehmen können. Die unter üblichen Lastbedingungen auftretenden Zugkräfte müssen dabei von den Bändern und Muskeln aufgenommen werden. Als weiterer Nachteil von bekannten Gelenkprothesen, insbesondere von solchen, deren Gelenksflächen mit selbstschmierenden Kunststoffen beschichtet sind, kann angeführt werden, dass diese eine beschränkte Lebensdauer aufweisen, d.h. dass die Gelenksflächen einem Verschleiss unterliegen.

Aus der EP-0 310 483 ist eine Fingergelenkprothese, insbesondere Metacarpophalangial- oder Interphalangialprothese, bekannt, welche aus zwei zusammenwirkenden Elementen, die das eigentliche Gelenk bilden, besteht. Beide Elemente sind mit einem in den Knochenmarkkanal implantierbaren Schaft versehen. Das eine Element weist auf der einen Seite die Form eines in einer Ebene offenen Gehäuses auf, währenddem das andere Element einen Kopf besitzt, der in dem Gehäuse aufgenommen werden kann. Das Gehäuse ist auf seiner Aussenseite mit zwei Ränder versehen, auf welchen sich der Kopf mit zwei konkaven Aussparungen beweglich abstützen kann. Der Kopf ist zudem so ausgebildet, dass er zwei ebene Seitenflächen besitzt, deren Abstand etwas geringer als die Öffnung des Gehäuses ist, wodurch der Kopf bzw. dessen Seitenflächen im Gehäuse so geführt wird/werden, dass die beiden Elemente hauptsächlich in einer Ebene bewegbar sind. Bei einer bevorzugten Ausführungsform ist zudem vorgesehen, dass die Fingergelenkprothese in gestrecktem Zustand gewisse seitliche Bewegungen zulässt.

Nachteilig bei einer derart ausgebildeten Fingergelenkprothese ist, dass von ihr selber nur Druckkräfte aufgenommen werden können. Die Erfahrung zeigt jedoch, dass bei Patienten, denen eine Fingergelenkprothese eingesetzt wird bzw. werden sollte, die Bänder sehr oft nicht mehr die gewünschte Stabilität aufweisen. Daher ist die Gefahr gross, dass eine derartig ausgebildete Gelenkprothese schon bei relativ geringen Zug-Kräften, wie sie täglich auftreten können, luxiert. Somit wäre es wünschenswert, wenn die Gelenkprothese selber die durch übliche Belastungen auftretenden Zug-Kräfte aufnehmen könnte. Als Beispiel für Leute mit stark geschwächten Bändern seien Patienten mit Rheumatismus aufgeführt. Ein weiterer Nachteil dieser Gelenkprothese besteht darin, dass die Fingergelenkprothese nur in gestrecktem Zustand seitliche Bewegungen zulässt, wobei keine eigentlichen Gelenksflächen für eine seitliche Bewegung vorhanden sind.

Aus der EP 0 280 424 ist zudem eine Gelenkprothese bekannt, bei welcher die beiden Prothesenhälften über Mittel zum Zusammenkoppeln derselben im Bereich der Drehachse verfügen. Eine solche Gelenkprothese kann gewisse Zugkräfte aufnehmen, hat jedoch andererseits den Nachteil, dass die beiden Prothesenhälften nur in einer Ebene relativ zueinander bewegbar sind. Ausserdem ist der Aufbau einer solchen Gelenkprothese komplex, wodurch sie in der Herstellung teuer ist. Im weiteren ist die Gelenkprothese im Bereich des eigentlichen Gelenks unnatürlich gross ausgebildet, was zu Schwierigkeiten beim Einsetzen bzw. zu nachfolgenden Komplikationen führen kann.

Ein weiterer Nachteil der bekannten Gelenkprothesen, die nur in einer Ebene bewegbar sind, anhaftet, besteht darin, dass seitlich, ausserhalb der Gelenkprothesen-Bewegungsebene einwirkende Kräfte grosse Momente und Scherkräfte verursachen können, welche die Gelenkprothese über das vorgesehene Mass hinaus belasten und dadurch zu nachhaltigen Schäden an der Gelenkprothese führen und diese eventuell sogar zerstören können.

Es ist daher die Aufgabe der Erfindung, eine Gelenkprothese der hier zur Rede stehenden Art derart zu verbessern, dass diese in implantiertem Zustand auf Druck und auf Zug belastbar ist und eine grössere Bewegungsfreiheit bei gleichzeitig minimierter Luxationsgefahr aufweist, wobei die beiden Prothesenhälften leicht zusammenfügbar sein sollen.

Diese Aufgabe wird durch eine Gelenkprothese gelöst, welche die im Anspruch 1 aufgeführten Merkmale besitzt.

Das Grundprinzip der erfindungsgemässen, aus zwei Prothesenhälften bestehenden Gelenkprothese beruht darauf, dass die beiden Prothesenhälften derart ausgebildet sind, dass sie in einer Montagestellung zusammengefügt und danach in eine Wirkstellung ausgerichtet werden können, in welcher die eine Prothesenhälfte gegenüber der anderen sowohl auf Druck wie auch auf Zug belastbar ist. Dazu weist die erste Prothesenhälfte Abflachungen am Kopf auf, währenddem die andere Prothesenhälfte eine kugelkalottenförmig ausgestaltete Pfanne aufweist, deren Höhe grösser als der Kugelradius ist und deren kugelkalottenförmige Gestaltung sich in der Höhe über den Kugelradius hinaus erstreckt. Um die beiden Prothesenhälften in einer Montagestellung zusammenfügen zu können, ist die Pfanne mit zumindest einem Ausschnitt versehen. Nach dem Zusammenfügen wird die Gelenkprothese in ihre von der Montagestellung abweichende Wirkstellung gebracht, in welcher sie auf Zug belastbar ist.

Bevorzugte Ausführungsformen der Erfindung sind in den abhängigen Ansprüchen 2 bis 16 umschrieben.

So ist bei einer bevorzugten Ausführungsform vorgesehen, dass die am Kopf der ersten Prothesenhälfte angebrachten Abflachungen in der Wirkstellung der Gelenkprothese mit der Hauptbeugeebene einen Winkel von 10° bis 80° einschliessen. Dieser Winkel, der nach dem Zusammenfügen und anschliessenden Verdrehen der einen gegenüber der anderen Prothesenhälfte besteht, ist dabei so gewählt, dass er grösser ist, als die Physilogie des entsprechenden Gelenks dies unter normalen Umständen zulassen würde. Der Verdrehwinkel kann somit je nach Einsatzort des Gelenks den physiologischen Vorgaben angepasst und bei der Fertigung des Gelenks berücksichtigt werden. Auf diese Weise wird sichergestellt, dass die Gelenkprothese, unter den üblichen, auftretenden Belastungen, nicht luxiert, da dazu die eine Prothesenhälfte gegenüber der anderen um den vorgängig definierten Winkel verdreht werden müsste. Beispielsweise könnte der Verdrehwinkel bei einem Fingermittengelenk relativ klein gewählt werden währenddem er bei einem Schultergelenk eher gross gewählt werden müsste.

Bei einer weiteren, bevorzugten Ausführungsform der Gelenkprothese ist vorgesehen, dass der Kopf der ersten Prothesenhälfte gegenüber der Längsmittelachse des Schafts geneigt ist. Dies ermöglicht, die erste Prothesenhälfte in der einen Richtung stärker auszulenken als dies bei einem ohne Neigung angebrachten Kopf der Fall wäre.

Eine weitere, bevorzugte Ausführungsform sieht zudem vor, dass in die kugelkalottenförmig ausgestaltete Pfanne eine der Aufnahme von Gelenkflüssigkeit dienende Vertiefung eingelassen ist. Dies gewährleistet eine zuverlässige Schmierung der Gelenksflächen. Ist die Gelenksoberfläche des Kopfs zudem noch, wie in einer weiteren, bevorzugten Ausführungsform vorgesehen, mit einer Vielzahl von Vertiefungen versehen, so begünstigt dies die Schmierung zusätzlich.

Nachfolgend wird eine Ausführungsbeispiel der Erfindung anhand von Zeichnungen näher erläutert. In den Zeichnungen zeigt:
Fig. 1 eine Ansicht der ersten Prothesenhälfte, welche mit einem im wesentlichen kugelförmigen Kopf versehen ist;
Fig. 2 eine Ansicht der Prothesenhälfte der Fig. 1, wobei diese gegenüber der Fig. 1 um 90° um ihre Längsachse verdreht ist;
Fig. 3 eine Ansicht auf die Prothesenhälfte der Fig. 1 und 2 vom Schaft her;
Fig. 4. die zweite Prothesenhälfte mit kugelkalottenförmig ausgestalteter Pfanne in teilweisem Querschnitt;
Fig. 5 einen teilweisen Längsschnitt durch die Prothesenhälfte der Fig. 4 in einer um 90° um die Längsachse verdrehten Ansicht;
Fig. 6 einen Querschnitt durch die Pfanne der zweiten Prothesenhälfte;
Fig. 7 eine erste Prinzipdarstellung zur Erläuterung der Funktionsweise der Gelenkprothese;
Fig. 8 eine zweite Prinzipdarstellung zur Erläuterung der Funktionsweise der Gelenkprothese;
Fig. 9 eine dritte Prinzipdarstellung zur Erläuterung der Funktionsweise der Gelenkprothese, und
Fig. 10 die Gelenkprothese in zusammengefügtem Zustand.

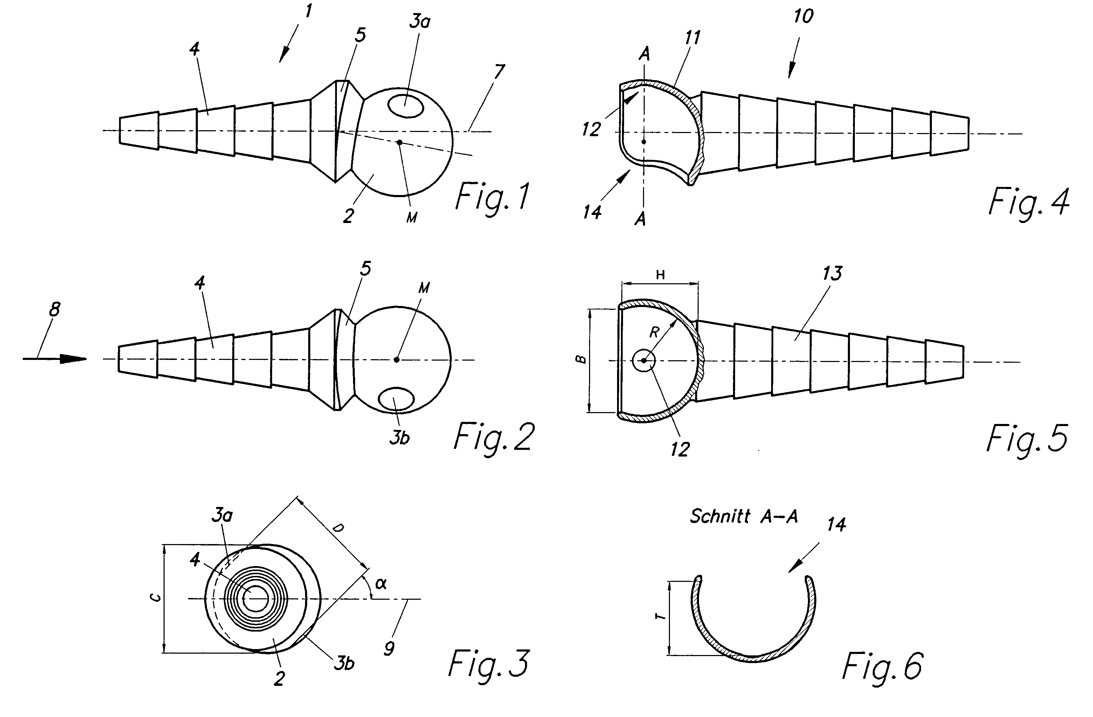

In den Fig. 1 und 2 ist die erste der beiden Prothesenhälften in einer Draufsicht dargestellt, wobei diese Prothesenhälfte 1 in der Fig. 2. gegenüber der Fig. 1 in einer um 90° um die Längsachse verdrehten Position eingezeichnet ist. Die Prothesenhälfte 1 weist einen im wesentlichen kugelförmigen Kopf 2 und einen damit verbundenen Verankerungsschaft 4 auf. Der Verankerungsschaft 4 besitzt eine kegelförmige Grundform und weist eine Mehrzahl von Einkerbungen auf. Der Kopf 2 ist zudem mit zwei einander diametral gegenüberliegenden Abflachungen 3a, 3b versehen. Aus der Fig. 1 ist ersichtlich, dass das Kopfzentrum M nicht auf der Längsmittelachse 7 des Schaftes 4 liegt, sondern dass das Kopfzentrum M in Bezug auf die Längsmittelachse 7 versetzt ist. Ausserdem ist der Kopf 2 über ein halsförmiges, asymmetrisches Zwischenstück 5 so mit dem Schaft 4 verbunden, dass dieser gegenüber der Längsmittelachse 7 und damit gegenüber dem Verankerungsschaft 4 in einer Ebene geneigt ist.

In der Fig. 3 ist die erste Prothesenhälfte 1 in einer Ansicht vom Schaft 4 her dargestellt, wie dies durch den Pfeil 8 in Fig. 2 angedeutet ist. Aus dieser Darstellung ist ersichtlich, dass die beiden Abflachungen 3a, 3b derart am Kopf 2 angebracht sind, dass sie mit der dem Kopf 2 und dem Schaft 4 gemeinsamen Zentralmittelebene 9 einen Winkel α von 45° einschliessen. In der Wirkstellung der Gelenkprothese, welche nachfolgend noch erläutert wird, fällt die Zentralmittelebene 9 mit der Hauptbeugeebene der Gelenkprothese zusammen.

Die Oberfläche des Kopfs 2 weist eine Vielzahl mikroskopisch kleiner Vertiefungen auf, welche die Schmierung der beiden Gelenkprothesen 1, 10 unterstützen. In Versuchen hat sich gezeigt, dass sehr gute Ergebnisse erzielt werden, wenn eine Mehrzahl dieser Vertiefungen einen Durchmesser zwischen 1 und 5 Mikrometer und eine Tiefe zwischen 1 und 5 Mikrometer aufweisen.

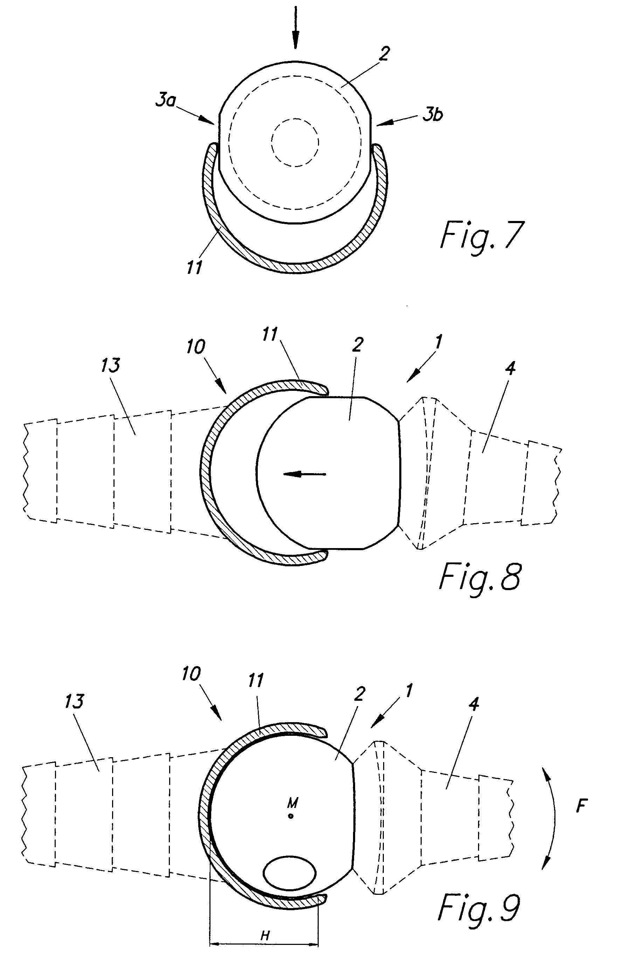

In den Fig. 4 bis 6 ist die zweite Prothesenhälfte 10, in teilweise geschnittenem Zustand, dargestellt. Diese Prothesenhälfte 10 weist eine kugelkalottenförmig ausgestaltete, einstückige Pfanne 11 sowie einen damit verbundenen Verankerungsschaft 13 auf. In der Fig. 4 ist die Pfanne 11 in einem ersten Längsschnitt und in der Fig. 5 in einem zweiten, um 90° verdrehten Längsschnitt dargestellt, währenddem aus der Fig. 6 die Pfanne 11 in einem Querschnitt entlang der Linie A-A in Fig. 4 ersichtlich ist. Die Innenfläche der Pfanne 11 ist korrespondierend zum Kopf 2 der ersten Prothesenhälfte 1 ausgestaltet, so dass dieser beweglich darin aufgenommen werden kann. Aus der Fig. 5 ist ersichtlich, dass die Höhe H der Pfanne 11 grösser ist als der Kugelradius R, und dass sich die kugelkalottenförmige Gestaltung der Pfanne 11 in der Höhe H über den Kugelradius R hinaus erstreckt. Dadurch kann die Pfanne 11, im zusammengefügten Zustand der beiden Prothesenhälften 1, 10, den Kopf 2 über dessen Kopfzentrum M hinaus umschliessen.

Um den Kopf 2 in die Pfanne 11 einzuführen, weist diese auf der einen Seite einen Ausschnitt 14 auf, der so bemessen ist, dass die verbleibende Tiefe T der Pfanne 11, zwischen der Vorderkante und dem Pfannenzentrum, grösser als deren Radius R ist. Aus der Fig. 6 ist zudem ersichtlich, dass sich die kugelkalottenförmige Gestaltung der Pfanne 11 auch in der Tiefe T über den Kugelradius R hinaus erstreckt.

Die Breite B des Ausschnitts 14 im Bereich der Stirnkante der Pfanne 11 ist kleiner als der Kopfdurchmesser C ausserhalb der Abflachungen 3a, 3b und grösser als der Abstand D zwischen den beiden Abflachungen 3a, 3b. Auf der Innenseite der Pfanne 11 ist zudem eine Vertiefung 12 eingelassen, welche der Aufnahme von Gelenkflüssigkeit dient und dadurch die Schmierung der Gelenksflächen unterstützt.

Die beiden Prothesenhälften 1, 10 sind im wesentlichen aus Titan oder einer Titanlegierung gefertigt. Im Bereich der Gelenksflächen sind die Prothesenhälften durch Ostid-Materialien, Titan-Niob- und Titan-Zirkonium-Keramik, veredelt, was zu einer extremen Oberflächenhärte im Bereich von 25000 HV (Vickers-Härte in N/mm²) führt. Diese hohe Härte erlaubt, dass die Gelenkprothese im Bereich der Gelenksflächen nicht mit selbstschmierenden Werkstoffen beschichtet werden muss, sondern dass die Schmierung durch die natürliche, zur Verfügung stehende Gelenkflüssigkeit erfolgen kann. Im weiteren wird durch diese hohe Härte garantiert, dass selbst während einer Einsatzdauer von mehereren Jahren kein messbarer Verschleiss auftritt.

Die Verankerungsschäfte 4, 13 beider Prothesenhälften 1, 10 sind mit Hydroxylapatit, insbesondere Hydroxylapatit-Keramik, beschichtet, wodurch ein Anwachsen des Knochens an den jeweiligen Schaft gefördert wird. Der Verankerungsschaft 13 der zweiten Prothesenhälfte 10 ist länger und im Durchmesser grösser als der Verankerungsschaft 4 der ersten Prothesenhälfte 1 ausgebildet. Dadurch eignet sich die erste Prothesenhälfte 1 bevorzugt zum distalen Verankern, währenddem die zweite Prothesenhälfte 10 zum proximalen Verankern geeignet ist. Verankerungsschäfte der hier zur Rede stehenden Art sind bekannt, weshalb an dieser Stelle nicht näher darauf eingegangen zu werden braucht.

Anhand der Figuren 7 bis 9 soll das Prinzip der vorgängig beschriebenen Gelenkprothese näher erläutert werden. Dazu sind die beiden Prothesenhälften 1, 10 in auf das wesentliche beschränkten Ansichten dargestellt. Die beiden Verankerungsschäfte 4, 13 sind schematisch durch unterbrochene Linien angedeutet. In der Fig. 7 ist die Pfanne 11 der zweiten Prothesenhälfte 10 in einem Querschnitt und der Kopf 2 der ersten Prothesenhälfte 1 in einer Ansicht vom Schaft her, dargestellt. In den Fig 8 und 9 ist die Pfanne 11 in einem Längsschnitt und der Kopf 2 in einer Draufsicht dargestellt, wobei die mit dem Kopf 2 versehene Prothesenhälfte 1 in der Fig. 9 gegenüber der Fig. 8 um 45° um ihre Längsachse verdreht ist.

Um den Kopf 2 in die Pfanne 11 einzuführen, müssen die beiden Prothesenhälften 1, 10 in die Montagestellung ausgerichtet werden, welche in den Fig. 7 und 8 in zwei unterschiedlichen Ansichten dargestellt ist. Dazu werden die Abflachungen 3a, 3b, wie in der Fig. 7 dargestellt, vertikal ausgerichtet, so dass diese gegen den sich über den Mittelpunkt hinaus erstreckenden Bereich der Pfanne 11 gerichtet sind. In dieser Montagestellung wird das Zusammenfügen der beiden Prothesenhälften 1, 11 ermöglicht, wobei die beiden Prothesenhälften 1, 11 in dieser Stellung sowohl zusammengefügt wie auch auseinander genommen werden können. Die Gelenksfläche der Pfanne 11 dient beim Zusammenfügen der beiden Prothesenhälften 1, 10 gleichzeitig als Anschlag für den Kopf 2 der ersten Prothesenhälfte 1.

In der Fig. 9 ist die Gelenkprothese schliesslich noch im zusammengefügten Zustand in der Wirkstellung dargestellt. Aus dieser Figur ist ersichtlich, dass die Pfanne 11 den Kopf 2 über den Kopfmittelpunkt M hinaus umschliesst. Um in die Wirkstellung zu gelangen, wurde die erste Prothesenhälfte 1, nach dem in Fig. 8 dargestellten Zusammenfügen, um 45° verdreht, wodurch sie nun bewegbar in der Pfanne 11 aufgenommen und von dieser über den Kopfmittelpunkt M hinaus umschlossen wird. In dieser Stellung fällt die Zentralmittelebene 9 der ersten Gelenkprothese 1 mit der Hauptbeugeebene der Gelenkprothese zusammen, wodurch die eine Prothesenhälfte gegenüber der anderen in einem Arbeitsbereich bewegt und dabei auf Zug belastet werden. Bei der Bewegung der ersten 1 gegenüber der zweiten Prothesenhälfte 10 dienen die Pfannenränder gleichzeitig als Anschlag für die erste Prothesenhälfte 1. Das einzige Kriterium bezüglich der Zugbelastung ist, dass die beiden Prothesenhälften 1, 10, bezogen auf ihre Längsachsen, nicht gegeneinander um 45° bzw. 135° in die Montagestellung zurück verdreht werden dürfen. Solange dies nicht geschieht, kann die Gelenkprothese auch nicht luxieren. Dies ist jedoch durch die Physiologie des menschlichen Körpers in den allermeisten Fällen auch nicht möglich. Die Auslenkbarkeit der ersten 1 gegenüber der zweiten Prothesenhälfte 10 in der einen Ebene ist in der Fig. 9 durch Pfeile F angedeutet.

Das Verankern der jeweiligen Prothesenhälften 1, 10 in je einem der Gelenkprothese zugewandten Knochen und das anschliessende Zusammenfügen der beiden Prothesenhälften 1, 10 soll nachfolgend erläutert werden. Dies soll am Beispiel des Einsetzens der Gelenkprothese als Fingergrundgelenk geschehen. Dazu wird die mit der Pfanne 11 versehene Prothesenhälfte 10 proximal im Metacarpus und die mit dem Kopf 2 versehene Prothesenhälfte 1 distal in der Phalanx I, in bekannter Weise, verankert. Dabei werden die beiden Prothesenhälften in ihre Wirkstellung (Fig. 9) ausgerichtet, wobei die beiden Prothesenhälften 1, 10 noch nicht zusammengefügt sind. Danach wird die in die Phalanx I eingesetzte Prothesenhälfte 1 an dem sie umschliessenden Finger soweit verdreht, bis die beiden Prothesenhälften 1, 10 zueinander in der Montagestellung zu liegen kommen. Dazu muss im vorliegenden Fall der Finger um 45° verdreht werden. Nun werden die beiden Prothesenhälften 1, 10 zusammengefügt indem der Kopf 2 der einen Prothesenhälfte 1 in die Pfanne 11 der anderen Prothesenhälfte 10 eingeführt wird. Anschliessend wird der Finger in seine Ausgangsstellung zurückgedreht wodurch die Gelenkprothese ihre Wirkstellung einnimmt und theoretisch einsatzbereit ist. Nun können in bekannter Weise die Bänder, Sehnen und Muskeln in die richtige Lage gebracht werden.

Nachdem die Knochen am jeweiligen Verankerungsschaft angewachsen sind, ist die Gelenkprothese voll belastbar, wobei die Gefahr einer Luxation minimiert ist bzw. praktisch ausgeschlossen werden kann, da diese nur dann eintreten kann, wenn der Finger um 45° verdreht und dabei auf Zug belastet wird.

Die Schmierung der Gelenkprothese erfolgt über die natürliche Gelenkflüssigkeit. Die in die kugelkalottenförmig ausgestaltete Pfanne 11 eingelassene Vertiefung 12 stellt zudem die Schmierung sicher, indem diese als Reservoir für die natürliche Gelenkflüssigkeit dient.

Durch die extreme Härte der Gelenksoberflächen entsteht keine messbare Abnutzung.

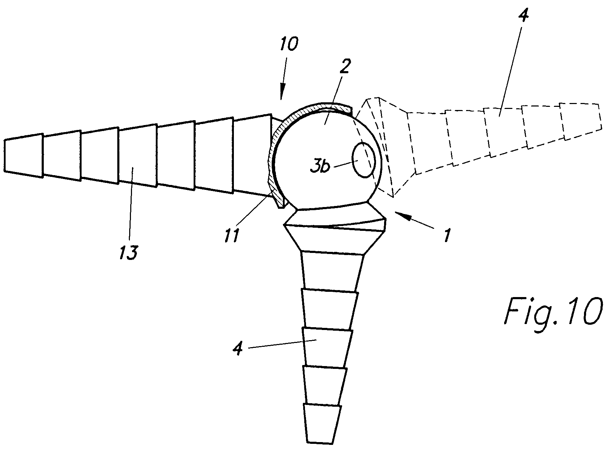

Aus der Fig. 10 ist schliesslich noch die Gelenkprothese in zusammengesetztem Zustand in einer Seitenansicht dargestellt. Der maximale Bewegungsweg in der Hauptbeugeebene ist durch die in der Nähe der beiden Endpositionen eingezeichnete erste Prothesenhälfte 1 angedeutet, wobei die gestreckte Position der ersten Prothesenhälfte 1 durch unterbrochene Linien angedeutet ist. Wie aus dieser Darstellung ersichtlich ist, hat ein mit Neigung am Schaft 4 angebrachter Kopf 2 den Vorteil, dass die erste Prothesenhälfte 1 in der einen Richtung, im vorliegenden Ausführungsbeispiel nach unten, stärker ausgelenkt werden kann, als dies bei einem zentrisch angeordneten Kopf 2 der Fall wäre. Die Pfannenränder der zweiten Prothesenhälfte 10 dienen als Anschlag für die Auslenkbewegung der ersten Prothesenhälfte 1.

Zu erwähnen ist, dass die vorgängig umschriebene Gelenkprothese lediglich ein mögliches Ausführungsbeispiel darstellt. Es sind noch eine Vielzahl anderer Ausführungsformen von erfindungsgemässen Gelenkprothesen denkbar, bei denen beispielsweise der Versatz der Abflachungen, die Höhe der Pfanne, die Grösse der Abflachungen, die maximale Auslenkbarkeit der einen gegenüber der anderen Prothesenhälfte sowie auch die Anordnung der Prothesenhälfte, distal und proximal, variieren können. Im weiteren könnten, anstelle eines Ausschnitts 14, auch zwei einander gegenüber liegende Ausschnitte angebracht werden. Auch bezüglich der Materialwahl und der Oberflächenveredelung im Bereich der Gelenksflächen sind andere Varianten denkbar, wobei anzufügen ist, dass die vorgängigen Aufzählungen keinesfalls als abschliessend zu betrachten sind.

## Patentansprüche

1. Aus zwei zusammenfügbaren, zusammenwirkenden Prothesenhälften (1, 10) bestehende Gelenkprothese, bei der die beiden Prothesenhälften (1, 10) je einen Verankerungsschaft (4, 13) zum jeweiligen Verankern in einem der Gelenkprothese zugewandten Knochen aufweisen und die erste Prothesenhälfte (1) mit einem im wesentlichen kugelförmigen Kopf (2) versehen ist, der zum Zusammenfügen mit der zweiten Prothesenhälfte (10) diametral gegenüberliegende Abflachungen (3a, 3b) aufweist, wobei die zweite Prothesenhälfte (10) eine im wesentlichen kugelkalottenförmig ausgestaltete Pfanne (11) aufweist, deren Höhe (H) grösser ist als der Kugelradius (R) und wobei sich die kugelkalottenförmige Gestaltung der Pfanne (11) in der Höhe (H) über den Kugelradius (R) hinaus erstreckt, wobei die Pfanne (11) ferner mit einem Ausschnitt (14) zum Zusammenfügen der beiden Prothesenhälften (1, 10) in einer Montagestellung versehen ist, dessen Breite (B) im Bereich der Stirnkante der Pfanne (11) kleiner ist als der Kopfdurchmesser (C) ausserhalb der Abflachungen (3a, 3b) und grösser als der Abstand (D) zwischen den beiden Abflachungen (3a, 3b), so dass die Gelenkprothese im zusammengefügten Zustand von einer Montagestellung in eine Wirkstellung verdrehbar ist, in der die Gelenkprothese auf Zug belastbar ist.

2. Gelenkprothese nach Anspruch 1, dadurch gekennzeichnet, dass der Ausschnitt derart ausgebildet ist, dass die eine Prothesenhälfte gegenüber der anderen zumindest in einer Ebene um mehr als 80° auslenkbar ist.

3. Gelenkprothese nach Anspruch 2, dadurch gekennzeichnet, dass die Ebene, in der die eine gegenüber der anderen Prothesenhälfte (1, 10) um mehr als 80° auslenkbar ist, der Hauptbeugeebene entspricht, und dass die am Kopf (2) der ersten Prothesenhälfte (1) angebrachten Abflachungen (3a, 3b) in der Wirkstellung der Gelenkprothese mit der Hauptbeugeebene einen Winkel einschliessen.

4. Gelenkprothese nach Anspruch 3, dadurch gekennzeichnet, dass die am Kopf (2) der ersten Prothesenhälfte (1) angebrachten Abflachungen (3a, 3b) in der Wirkstellung der Gelenkprothese mit der Hauptbeugeebene einen Winkel von 10° bis 80° einschliessen.

5. Gelenkprothese nach einem der vorhergehenden Ansprüche, dadurch gekennzeichnet, dass die Gelenkprothese im zusammengesetzten Zustand drei Freiheitsgrade, zwei translative und einen rotativen, aufweist.

6. Gelenkprothese nach einem der vorhergehenden Ansprüche, dadurch gekennzeichnet, dass der Mittelpunkt (M) des Kopfs (2) der ersten Prothesenhälfte (1) gegenüber der Längsmittelachse (7) des Schafts (4) versetzt ist.

7. Gelenkprothese nach einem der vorhergehenden Ansprüche, dadurch gekennzeichnet, dass der Kopf (2) gegenüber der Längsmittelachse (7) des Schafts (4) geneigt und über ein halsförmiges Zwischenstück (5) mit dem Schaft (4) verbunden ist.

8. Gelenkprothese nach einem der Ansprüche 4 oder 5, dadurch gekennzeichnet, dass die Abflachungen (3a, 3b) am Kopf (2) mit einer dem Kopf (2) und dem Verankerungsschaft (4) der ersten Prothesenhälfte (1) gemeinsamen Längsmittelebene (9), einen Winkel (a) von 10° bis 80°, vorzugsweise 30 bis 60°, einschliessen.

9. Gelenkprothese nach Anspruch 8, dadurch gekennzeichnet, dass in der Wirkstellung der Gelenkprothese die Längsmittelebene (9) der ersten Prothesenhälfte (1) mit der Hauptbeugeebene der Gelenkprothese zusammenfällt.

10. Gelenkprothese nach einem der vorhergehenden Ansprüche, dadurch gekennzeichnet, dass die Ränder der kugelkalottenförmig ausgestalteten Pfanne (11) als Bewegungsanschlag für die erste Prothesenhälfte (1) ausgebildet sind und den Arbeitsbereich derselben begrenzen.

11. Gelenkprothese nach einem der vorhergehenden Ansprüche, dadurch gekennzeichnet, dass der Verankerungsschaft (4) der ersten, mit dem im wesentlichen kugelförmigen Kopf (2) versehenen Prothesenhälfte (1) zum distalen Verankern ausgebildet ist, und dass der Verankerungsschaft (13) der mit der kugelkalottenförmig ausgestalteten Pfanne (11) versehenen, zweiten Prothesenhälfte (10) zum proximalen Verankern ausgebildet ist.

12. Gelenkprothese nach einem der vorhergehenden Ansprüche, dadurch gekennzeichnet, dass in die kugelkalottenförmige Pfanne (11) eine der Aufnahme von Gelenkflüssigkeit dienende Vertiefung (12) eingelassen ist.

13. Gelenkprothese nach einem der vorhergehenden Ansprüche, dass sowohl die kugelkalottenförmig ausgestaltete Pfanne (11) der zweiten Prothesenhälfte (10) als der zumindest teilweise kugelförmige Kopf (2) der ersten Prothesenhälfte (1) im wesentlichen aus Titan oder einer Titanlegierung besteht, wobei die Oberflächen der Pfanne (11) und des Kopfs (2) zumindest im Bereich der Gelenksflächen eine Vickers-Härte von über 5000, vorzugsweise von über 20'000, aufweisen.

14. Gelenkprothese nach Anspruch 13, dadurch gekennzeichnet, dass die beiden Prothesenhälften (1, 10) zumindest im Bereich der Gelenksflächen mit Keramik Legierungen veredelt sind.

15. Gelenkprothese nach einem der vorhergehenden Ansprüche, dadurch gekennzeichnet, dass die Gelenksoberfläche des Kopfs (2) mit einer Vielzahl von Vertiefungen versehen ist, welche die Schmierung des Gelenks unterstützen, wobei eine Mehrzahl dieser Vertiefungen einen Durchmesser zwischen 1 und 5 Mikrometer und eine Tiefe zwischen 1 und 5 Mikrometer aufweisen.

16. Gelenkprothese nach einem der vorhergehenden Ansprüche, dadurch gekennzeichnet, dass die Verankerungsschäfte (4, 13) mit einer das Knochenwachstum fördernden Schicht, vorzugsweise Hydroxylapatit oder Hydroxylapatit-Keramik, beschichtet sind.
